# EUROPÄISCHE PATENTSCHRIFT

(11) **EP 1 628 684 B1**
(45) Veröffentlichungstag und Bekanntmachung des Hinweises auf die Patenterteilung: **21.04.2010**
(21) Anmeldenummer: 04735236.4
(22) Anmeldetag: 28.05.2004
(51) Int. Cl.: A61K 47/48

(54) **WASSERLÖSLICHE KOMPLEXE VON ARZNEISTOFFEN MIT HOCHMOLEKULAREN STÄRKEDERIVATEN**
WATER SOLUBLE COMPLEXES OF MEDICINAL SUBSTANCES WITH HIGH-MOLECULAR WEIGHT STARCH DERIVATIVES
COMPLEXES CONSTITUES DE SUBSTANCES MEDICAMENTEUSES ET DE DERIVES DE L'AMIDON DE MASSE MOLECULAIRE ELEVEE

(30) Priorität: 30.05.2003 DE 10324710
(43) Veröffentlichungstag der Anmeldung: 01.03.2006
(73) Patentinhaber: Supramol Parenteral Colloids GmbH, 61191 Rosbach-Rodheim (DE)
(72) Erfinder: SOMMERMEYER, Klaus, 61191 Rosbach v.d.H. (DE)
(74) Vertreter: Luderschmidt, Schüler & Partner
(86) Internationale Anmeldenummer: PCT/EP2004/005801
(87) Internationale Veröffentlichungsnummer: WO 2004/105800

(56) Entgegenhaltungen:
- EP-A- 1 106 186
- WO-A-02/02146
- WO-A-99/51284
- WO-A-02/080979
- WO-A-03/000738
- WO-A-03/074088
- KAYSER O. ET AL: "Formulation and biopharmaceutical issues in the development of drug delivery systeims for antiparasitic drugs" PARASITOL. RES., Bd. 90, 11. Dezember 2002 (2002-12-11), Seiten S63-S70, XP008039883
- JAE-YOUNG J. ET AL.: "Enhanced solubility and dissolution rate of itraconazole by a solid dispersion technique" INTERNATIONAL JOURNAL OF PHARMACEUTICS, Bd. 187, 1999, Seiten 209-218,

## Beschreibung

Die vorliegende Erfindung betrifft Komplexe aus einem Arzneistoff und einem hochmolekularen Träger sowie Injektions- und Infusionslösungen, die diese Komplexe aufweisen. Die Erfindung betrifft ferner Verfahren zur Herstellung solcher Injektions- und Infusionslösungen indem man den hochmolekularen Träger funktionalisiert, mit dem Arzneistoff umsetzt und in einer wässrigen Lösung löst.

Die kovalente chemische Bindung von pharmazeutischen Arzneistoffen an hochmolekulare Träger, wie zum Beispiel Polysaccharide, Proteine oder Polyethylenglycole, ist ein bekanntes Verfahren, um beispielsweise die Wasserlöslichkeit von an sich wasserunlöslichen oder schwer löslichen Verbindungen zu erhöhen. Solche Konjugate können auch vorteilhafterweise infolge der Vergrößerung des Molekulargewichts des pharmazeutischen Wirkstoffes im Vergleich zum underivatisierten Arzneistoff erheblich verlängerte Plasmahalbwertszeiten aufweisen. Auch ist es möglich, durch diese Technik die Antigenität therapeutischer Proteine herabzusetzen und so deren immunogene Nebenwirkungen zu reduzieren (vergleiche Abuchowski und Davis, Enzymes as drugs, Holcenberg und Rubberts, Herausgeber, S. 367-383, John Wiley & Sons N. Y. (1981))

Beispiele solcher Konjugate sowie deren Vorteile gegenüber den isolierten Arzneistoffen sind unter anderem in WO 98/01158, WO 03/000738 A2, WO02/080979, WO 97/33552 und WO 97/38727 beschrieben.

Obwohl mittlerweile solche kovalenten Kopplungsprodukte auf Basis von Polysacchariden oder auch auf Basis von Polyethylenglykolderivaten weit verbreitet sind, bleibt ein wesentlicher Nachteil dieser Technologie zu verzeichnen.

Durch die kovalente Verknüpfung eines Arzneistoffs mit einem Makromolekül, wie zum Beispiel Hydroxyethylstärke oder Polyethylenglykol, entsteht eine neue chemische Verbindung, die ebenfalls ein Arzneistoff ist. Diese muss aus arzneimittelrechtlichen Gründen erneut auf ihre Unbedenklichkeit und Wirksamkeit einer relativ aufwendigen, kostenintensiven und langwierigen Prüfung hinsichtlich ihrer Sicherheit und Wirksamkeit unterzogen werden, auch wenn der Arzneistoff an sich in dieser Beziehung gut charakterisiert ist.

Die Druckschrift Kayser et al. Formulation and biopharmaceutical issues in the development of drug delivery systems for antiparasitic drugs Parasitol Res (2003) 90, 63-70 betrifft die kovalente Bindung von Pentamidin, welches zwei basische Amidingruppen pro Molekül enthält, an ein Polymethacrylat.

Die Druckschrift WO 99/51284 beschreibt nicht kovalente Trägermolekül-Hapten-Biokonjugate. Dabei ist das Hapten sehr gut wasserlöslich.

Die Druckschrift EP 1 106 186 A2 offenbart nicht kovalente Trägermolekül-Hapten-Biokonjugate. Dabei ist das Hapten sehr gut wasserlöslich.

Die Druckschrift Chowdary et al. Biopharmaceutical studies on solid dispersions of nalidixic acid in modified starches Drug Development and Industrial Pharmacy, 20 (19), 3015-3022 (1994) beschreibt nicht kovalente Komplexe zwischen unfunktionalisierter HES und einem schwer wasserlöslichen Molekül (Nalidixinsäure) zur Bindung des Arzneistoffes.

Der Artikel Chiou et al. Pharmaceutical Applications of Solid Dispersion Systems Jounal of Pharmaceutical Sciences September 1971, 60/9 beschäftigt sich mit pharmazeutischen Anwendungen von festen Dispersionen. Stärke oder Stärkederivate, welche nur eine funktionelle Gruppe zur nicht kovalenten Bindung eines Arzneimittels in jedem Molekül aufweisen, werden in dieser Druckschrift nicht erwähnt.

Kushida et al. *Improvement of Dissolution and Oral Absorption of ER-34122,* A *poorly water-soluble dual 5-Lipoxygenase*/*Cyclooxygenase Inhibitor with anti-inflammatory activity by preparing solid dispersion* Journal of Pharmaceutical Sciences, January 2002, 91/1 beschreibt die Verwendung von Hydroxypropylmethylcellulose als Träger für die Verbesserung der Auflösungsgeschwindigkeit von ER-34122 (einem Arzneimittel), die durch die Bildung einer festen Dispersion deutlich größer ist als bei einer physikalischen Mischung des reinen Arzneimittels. Stärke oder Stärkederivate, welche nur eine funktionelle Gruppe zur nicht kovalenten Bindung eines Arzneimittels in jedem Molekül aufweisen, werden in dieser Druckschrift nicht erwähnt.

Die Druckschrift Jung et al. Enhanced Solubility and dissolution rate of itraconazole by a solid dispersion technique Journal of Pharmaceutics, 187 (1999), 209-218 beschäftigt sich mit der Erhöhung der Löslichkeit von Itraconazol bei der Auflösung von sogenannten festen Dispersionen dieser Verbindung in Polymeren. Beschrieben wird dabei die Verwendung von Aminoalkylmethacrylat-Copolymeren, Polyvinylacetaldiethylaminoacetat, Polyoxyethylen-polyoxypropylen-Copolymeren, Polyvinylpyrrolidon (PVP), Polyethylenglykol 20.000 und von Hydroxymethylcellulose. Stärke oder Stärkederivate, welche nur eine funktionelle Gruppe zur nicht kovalenten Bindung eines Arzneimittels in jedem Molekül aufweisen, werden in dieser Druckschrift nicht erwähnt.

Daher ist es Aufgabe der Erfindung Arzneistoffe zu liefern, die die oben erwähnten Vorteile von kovalenten Koplungsprodukten bestehend aus Arzneistoffen und polymeren Trägersubstanzen, aufweisen, die aber nicht einer solch aufwendigen Prüfung unterzogen werden müssen.

Weiter ist es Aufgabe der Erfindung Arzneistoffe zu liefern, die die oben erwähnten Vorteile von kovalenten Koplungsprodukten, bestehend aus Arzneistoffen und polymeren Trägersubstanzen, aufweisen, die aber kostengünstiger herstellbar sind.

Ebenso ist es Aufgabe der Erfindung Infusionslösungen und Injektionslösungen zu liefern, die Arzneistoffe aufweisen, die die oben erwähnten Vorteile von kovalenten Koplungsprodukten aus Arzneistoffen und polymeren Trägersubstanzen aufweisen, aber nicht einer solch aufwendigen Prüfung unterzogen werden müssen.

Eine weitere Aufgabe der Erfindung ist es, ein Verfahren zur Verlegung zu stellen, mit Hilfe dessen Verbindungen hergestellt werden, die die oben angegebenen Aufgaben lösen.

Überraschenderweise können die oben genannten erfindungsgemäßen Aufgaben durch molekulare Komplexe aus einer hochmolekularen Trägersubstanz und einem Arzneistoff gelöst werden, wobei die Trägersubstanz funktionelle Gruppen aufweist, wodurch die Arzneistoffe in einem Komplex nicht kovalent gebunden sind,
wobei
- der Arzneistoff in Wasser unlöslich oder schwer löslich ist,
- die Trägersubstanz in Wasser gut löslich ist,
- die Trägersubstanz ein Stärkederivat ist und nur eine funktionelle Gruppe zur nicht kovalenten Bindung des Arzneistoffs in jedem Molekül aufweist, wobei die funktionelle Gruppe eine saure oder basische Gruppe ist,
- der Komplex wasserlöslich ist
- die Trägersubstanz ein gewichtsgemitteltes Molekulargewicht im Bereich von 1.000 bis 1.000.000 Dalton aufweist,
- der Arzneistoff eine funktionelle Gruppe aufweist, die Komplexbildung ermöglicht, die aus einer basischen oder einer sauren Gruppe ausgewählt ist.

Solche nicht kovalenten Bindungen sind beispielsweise assoziative Bindungen, wie Wasserstoffbrückenbindungen, Salzbindungen oder mechanische Aggregate, die beispielsweise durch Verhakungen auf molekularer Ebene hervorgerufen werden. Erfindungsgemäße Komplexe liegen dann vor, wenn sich das Lösungsverhalten des Arzneistoffs in einem Lösungsmittel, in dem die hochmolekulare Trägersubstanz löslich ist, an das Lösungsverhalten der hochmolekularen Trägersubstanz anpasst, ohne dass beide Moleküle kovalent ineinander gebunden sind. Speziell bedeutet dies, dass sich die Löslichkeit eines nicht löslichen oder schwer löslichen Arzneistoffs verbessert, wenn die hochmolekulare Trägersubstanz in diesem Medium gut löslich ist. Vorzugsweise ist dieses Medium eine wässrige Lösung.

Vorzugsweise verbessert sich die Löslichkeit eines in Wasser nicht löslichen oder schwer löslichen Arzneistoffs bei Raumtemperatur durch die Zugabe einer hochmolekularen Trägersubstanz mindestens um den Faktor 100, besonders bevorzugt mindestens um den Faktor 1 000, insbesondere mindestens um den Faktor 10 000.

Das Gewichtsverhältnis von Arzneistoff und hochmolekularer Trägersubstanz ist nicht beschränkt. Vorzugsweise wird aber ein Verhältnis gewählt, beidem in einer Lösung des Komplexes eine pharmakologisch wirksame Menge an Arzneistoff gelöst ist und die Konzentration der hochmolekularen Trägersubstanz in einer solchen Lösung bevorzugt größer als 5 Gew. %, besonders bevorzugt größer als 10 Gew. % und insbesondere größer als 20 Gew. % ist.

Vorzugsweise sind sowohl die hochmolekulare Trägersubstanz als auch der Arzneistoff pharmakologisch unbedenklich und sind als Bestandteile von Arzneimitteln im Handel. Im Gegensatz zu den kovalent gebundenen Substanzen, die eine kostenintensive und langwierige Prüfung durchlaufen müssen, liegt bei erfindungsgemäßen Komplexen nur eine physikalische Mischung von an sich unbedenklichen Komponenten vor, so dass das Endprodukt nur noch einer reduzierten klinischen Sicherheitsprüfung unterzogen werden muss.

Unter einer hochmolekularen Trägersubstanz wird im Rahmen der Erfindung jedes einem Patienten verabreichbare Molekül mit einem gewichtsgemittelten Molekulargewicht im Bereich von 1.000 bis 1.000.000 Dalton, besonders bevorzugt im Bereich von 3.000 bis 300.000 Dalton und insbesondere von 6.000 bis 16.000 Dalton, verstanden.

Ganz besonders bevorzugt sind wasserlösliche Stärkederivate, insbesondere Hydroxyethylstärke. Hier wiederum ist HES bevorzugt.

HES ist das hydroxyethylierte Derivat des in Wachsmaisstärke zu über 95 % vorkommenden Glucosepolymers Amylopektin. Amylopektin besteht aus Glucoseeinheiten, die in α-1,4- glykosidischen Bindungen vorliegen und α-1,6-glykosidische Verzweigungen aufweisen. HES weist vorteilhafte rheologische Eigenschaften auf und wird zur Zeit als Volumenersatzmittel und zur Hämodilutionstherapie klinisch eingesetzt (Sommermeyer et al., Krankenhauspharmazie, Vol. 8 (8, 1987) Seite 271 - 278 und Weidler et. al., Arzneimittelforschung 1 Drug Res., 41, (1991) Seite 494- 498).

HES wird im wesentlichen über das gewichtsgemittelte mittlere Molekulargewicht M_{w}, das Zahlenmittel des mittleren Molekulargewichts Mₙ, die Molekulargewichtsverteilung und den Substitutionsgrad gekennzeichnet. Die Substitution mit Hydroxyethylgruppen in Etherbindung ist dabei an den Kohlenstoffatomen 2 (C2), 3 (C3) und 6 (C6) der Anhydroglucoseeinheiten möglich. Das Substitutionsmuster wird dabei als das Verhältnis der Substitution an C2 und C6 beschrieben (C2/C6-Verhältnis). Der Substitutionsgrad kann als DS ("degree of substitution"), welcher auf den Anteil der substituierten Glucosermoleküle aller Glucoseeinheiten Bezug nimmt, oder als MS ("molar substitution") beschrieben werden, womit die mittlere Anzahl von Hydroxyethylgruppen pro Glucoseeinheit bezeichnet wird.

In der wissenschaftlichen Literatur wird als Kurzbezeichnung für Hydroxyethylstärke das Molekulargewicht M_{w} in der Einheit kDalton zusammen mit dem Substitutionsgrad MS angegeben. So bezeichnet HES 10/0,4 eine Hydroxyethylstärke des gewichtsgemittelten Molekulargewichtes M_{w} von 10.000 Dalton und des Substitutionsgrades MS von 0,4.

Der molekulare Substitutionsgrad ist nicht beschränkt. Vorzugsweise liegt der molekulare Substitutionsgrad MS im Bereich von 0,05 bis 1,5, besonders bevorzugt im Bereich von 0,1 bis 0,8 und insbesondere im Bereich von 0,3 bis 0,5.

Das C2/C6-Verhältnis ist nicht beschränkt. Vorzugsweise ist dieses Verhältnis größer als 1, besonders bevorzugt ist dieses Verhältnis größer als 2 und insbesondere größer als 9.

Besonders bevorzugt ist ein erfindungsgemäßer Komplex, bei dem nur jeweils ein Molekül einer hochmolekularen Trägersubstanz an jeweils nur einen Arzneistoff durch nicht kovalente Bindung gebunden ist. Erfindungswesentlich ist, dass die hochmolekulare Trägersubstanz nur eine funktionelle Gruppe zur nicht kovalenten Bindung des Arzneistoffs in jedem Molekül aufweist.

Hierbei ist die funktionelle Gruppe eine saure oder eine basische Gruppe.

Weiterhin ist es bevorzugt, wenn die hochmolekulare Trägersubstanz die funktionelle Gruppe am vormals reduzierenden Ende aufweist.

Besonders bevorzugt ist dabei, dass die funktionelle Gruppe im Fall einer sauren funktionellen Gruppe, eine Aldonsäuregruppe ist.

Im Fall einer basischen funktionellen Gruppe ist eine primäre Aminogruppe bevorzugt. Weiterhin ist es besonders bevorzugt, dass die primäre Aminogruppe durch Konjugation eines aliphatischen Diamins über eine Amidbindung an eine Aldonsäuregruppe kovalent gebunden ist. Insbesondere bevorzugt ist, wenn sich diese Aldonsäuregruppe am vormals reduzierenden Ende der Polysaccharidkette befindet.

Die Bildung einer Aldonsäuregruppe am reduzierenden Ende eines Polysaccharids ist bekannt. Dies kann beispielsweise durch selektive Oxidation mit einem milden Oxidationsmittel, wie beispielsweise Hypo-Jodid, erfolgen.

Die Bildung einer primäre Aminogruppe durch Konjugation eines aliphatischen Diamins, das über eine Amidbindung an eine Aldonsäuregruppe kovalent gebunden ist, ist ebenso bekannt. Diese kann beispielsweise durch umsetzen des Diamins mit der Polysaccharid-Aldonsäure erfolgen.

Ein Beispiel für die Bildung einer Aldonsäuregruppe am reduzierenden Ende eines Polysaccharids ist die Herstellung von Hydroxyethylstärke -Aldonsäure ausgehend von Hydroxyethylstärke. So kann Beispielsweise nach WO 02/080979 Hydroxyethylstärke an der reduzierenden Endgruppe selektiv mit Hypo-Jodid zur Carbonsäure oxidiert werden, wodurch Hydroxyethylstärke-Aldonsäure erhalten wird.

Ein Beispiel für die Bildung einer Aminogruppe am reduzierenden Ende eines Polysaccharids ist die Herstellung von aminofunktionalisierter Hydroxyethylstärke, ausgehend von Hydroxyethylstärke -Aldonsäure. Dies ist in WO 02/080979 anhand der Umsetzung von Hydroxyethylstärke -Aldonsäure mit einem aliphatischen Diamin beschrieben.

Zur Bildung eines erfindungsgemäßen Komplexes können alle Arzneistoffe verwendet werden, die schlecht oder gar nicht wasserlöslich sind.

Bevorzugt sind Arzneistoffe die bereits eine arzneimittelrechtliche Zulassung besitzen. Allerdings müssen die Arzneistoffe, bereits eine funktionelle Gruppe aufweisen, die Komplexbindung ermöglicht, die aus einer basischen oder einer sauren Gruppe ausgewählt ist.

Eine besondere Ausführungsform der Erfindung stellen Komplexe von HES-Aldonsäure als hochmolekulare Komponente und einem Arzneistoff, ausgewählt ist aus der Gruppe, bestehend aus den Polyenmakrolid-Antibiotika Amphotericin B, Nystatin und Natamycin dar.

In dieser Ausführungsform weist HES-Aldonsäure vorzugsweise ein gewichtsgemitteltes Molekulargewicht von 1.000 bis 300.000 Dalton, einen molaren Substitutionsgrad von 0,1 bis 0,5 und ein C2/C6 Verhältnis von 2 bis 11 auf.

Besonders bevorzugt weist HES-Aldonsäure in dieser Ausführungsform ein gewichtsgemitteltes Molekulargewicht von 6.000 bis 16.000 Dalton, einen molaren Substitutionsgrad von 0,35 bis 0,45 sowie ein C2/C6-Verhältnis von 9 bis 11 auf.

Insbesondere sind in dieser Ausführungsform Komplexe einer solchen besonders bevorzugten HES-Aldonsäure mit dem Polyenmakrolid-Antibiotika Amphotericin B bevorzugt.

Eine besondere Ausführungsfonn der Erfindung stellen Komplexe von HES-Aldonsäure als hochmolekulare Komponente und dem Arzneistoff Antiarrhythmikum Amiodaron dar. Vorzugsweise weist HES-Aldonsäure in dieser Ausführungsform ein gewichtsgemitteltes Molekulargewicht von 1.000 bis 300.000 Dalton, einen molaren Substitutionsgrad von 0,1 bis 0,5 und ein C2/C6 Verhältnis von 2 bis 11 auf.

Besonders bevorzugt weist HES-Aldonsäure in dieser Ausführungsform ein gewichtsgemitteltes Molekulargewicht von 6.000 bis 16.000 Dalton, einen molaren Substitutionsgrad von 0,35 bis 0,45 sowie ein C2/C6-Verhältnis von 9 bis 11 auf.

Eine weitere, besondere Ausführungsform der Erfindung stellen Komplexe von HES-Aldonsäure als hochmolekulare Komponente und dem Arzneistoff Vancomycin dar. Vorzugsweise weist HES-Aldonsäure in dieser Ausführungsform ein gewichtsgemitteltes Molekulargewicht von 1.000 bis 300.000 Dalton, einen molaren Substitutionsgrad von 0,1 bis 0,5 und ein C2/C6 Verhältnis von 2 bis 11 auf.

Besonders bevorzugt weist HES-Aldonsäure in dieser Ausführungsform ein gewichtsgemitteltes Molekulargewicht von 6.000 bis 16.000 Dalton, einen molaren Substitutionsgrad von 0,35 bis 0,45 sowie ein C2/C6-Verhältnis von 9 bis 11 auf.

Ebenso ist es bevorzugt, dass der erfindungsgemäße Komplex einem Patienten parenteral applizierbar ist.

Durch Lösen erfindungsgemäßer Komplexe in einer geeigneten Trägerlösung können Injektionslösungen und Infusionslösungen erhalten werden. Durch Verwendung von Trägerlösungen zur Herstellung von erfindungsgemäßen Lösungen können Injektions- und Infusionslösungen erhalten werden. Vorzugsweise sind diese Trägerinfusionslösungen wässrige Lösungen. In einer besonderen Ausführungsform sind die wässrigen Trägerlösungen isotonische Lösungen, beispielsweise isotonische Glucoselösungen.

Die Herstellung der erfindungsgemäßen Komplexe kann durch Lösen der hochmolekularen Trägersubstanz und des Arzneistoffs in einer wässrigen Lösung, gegebenenfalls unter erwärmen, erfolgen. Vorzugsweise handelt es sich bei der wässrigen Lösung um eine isotonische Lösung. Um eine Oxidation der Komponenten zu verhindern wird vorzugsweise unter Schutzatmosphäre gearbeitet. Anschließend wird die erhaltene Lösung vorzugsweise filtriert, besonders bevorzugt steril filtriert.

Vorzugsweise ist die Konzentration des Arzneistoffs in einer solchen Lösung im pharmakologisch wirksamen Bereich. Die Konzentration des hochmolekularen Trägers ist nicht beschränkt. Vorzugsweise ist diese Konzentration aber größer als 5 Gew. %, besonders bevorzugt größer als 10 Gew. % und insbesondere größer als 20 Gew. %, bezogen auf die fertige Lösung.

Die Herstellung von erfindugsgemäßen Komplexen, bevorzugt im Fall von nicht wasserlöslichen Arzneistoffen, kann auch durch Lösen der hochmolekularen Komponente und des Arzneistoffs in einem gemeinsamen Lösemittel, in dem beide Komponenten löslich sind, erfolgen. Solche Lösemittel können beispielsweise polare, aprotische Lösemittel wie Dimethylsulfoxid (DMSO), Dimethylacetamid (DMA) oder Dimethylformamid (DMF) sein. Dabei wird gegebenenfalls unter Schutzgas gearbeitet, um eine Oxidation der Komponenten zu vermeiden. Nach Sterilfilrtation wird das Lösemittel unter schonenden Bedingungen, beispielsweise durch gefriertrocknen, entfernt.

Die so erhaltene lösemittelfreie Substanz kann beispielsweise als Ausgangsprodukt zur Herstellung eines Injektions- oder Infusionspräparats dienen.

Hierzu kann die so erhaltene, lösemittelfreie Substanz in einer wässrigen Lösung, beispielsweise in einem kleinen Volumen Wasser für Injektionszwecke und danach in einer kompatiblen Trägerinfusionslösung, wie beispielsweise 5 Gew. %-ige Glucoselösung, gelöst werden. Die Konzentration des löslichen Komplexes muss dabei so gewählt werden, dass die Lösung auch nach längerer Lagerung weder Trübung noch Partikelbildung noch sonstige Veränderungen zeigt, und so die Merkmale für injektabile Arzneiformen aufweist. Auch hier wird vorzugsweise unter Schutzgas gearbeitet.

Vorzugsweise ist die Konzentration des Arzneistoffs in einer solchen Lösung im pharmakologisch wirksamen Bereich. Die Konzentration des hochmolekularen Trägers nicht beschränkt. Vorzugsweise ist diese Konzentration aber größer als 5 Gew. %, besonders bevorzugt größer als 10 Gew. % und insbesondere größer als 20 Gew. %, bezogen auf die fertige Lösung.

Besonders bevorzugt ist dieses Verfahren zur Herstellung von erfindungsgemäßen Injektionslösungen und Infusionslösungen, die einen Komplexen aus HES-Aldonsäure und einem Polyenmakrolid-Antibiotikum aufweisen.

Die so hergestellten Lösungen der erfindungsgemäßen Komplexe stellen Ausgangssubstanzen für langzeitlagerstabile Fertigarzneimittel in Form von Injektions- oder Infusionslösungen dar, die ohne weitere Manipulation dem Patienten appliziert werden können.

In den folgenden Beispielen ist die Erfindung im Rahmen spezieller Ausführungsformen verdeutlicht. Hierdurch soll allerdings keine Beschränkung erfolgen.

### Beispiele

### Beispiel 1

**Herstellung von Amphotericin B - Komplexen mit HES 1010,4 - Aldonsäure.** 1 g HES 10/0,4 - Aldonsäure, welche nach Literaturvorschrift hergestellt wurde mit einem C2/C6-Verhältnis > 10, wurde in 10 mL Dimethylsulfoxid (DMSO) gelöst. Der Lösung werden 50 mg Amphotericin B gemäß Ph. Eur. zugefügt und nach dem kompletten Lösen wird der Ansatz über einen 0,45 µm Filter partikelfrei und steril filtriert.

Anschließend wird die Lösung gefriergetrocknet.

Das Lyophilisat löst sich glatt in 10 mL Wasser zu einer klaren, transparenten, gelb- orangefarbenen Lösung. Diese Lösung wird mit 5 Gew. %iger Glucoselösung zur Infusion auf 100 mL Gesamtvolumen weiterverdünnt. Die resultierende Lösung bleibt auch nach mehrmonatigem Lagern im Kühlschrank klar, transparent und partikelfrei.

### Vergleichsbeispiel 1

### Versuch der Herstellung eines Amphotericin B - HES 10/0,4 - Komplexes.

Die Versuchsdurchführung erfolgt wie im Beispiel 1 beschrieben, mit dem Unterschied, dass Anstelle der HES 10/0,4 -Aldonsäure HES 10/0,4 verwendet wurde. Nach dem Lyophilisieren löst sich jedoch das Produkt nicht mehr in 10 mL Wasser, sondern es verbleibt eine trübe, gelb-orange Suspension von Amphotericin B.

### Vergleichsbeispiel 2

### Versuch der Herstellung eines Gluconsäure - Amphotericin B - Komplexes.

Die Versuchsdurchführung erfolgt wie im Beispiel 1 beschrieben, mit dem Unterschied, dass Anstelle der HES 10/0,4 -Aldonsäure 1 g Glucose sowie die zu der in Beispiel 1 eingesetzten Menge HES 10/0,4 - Aldonsäure äquimolare Menge an Gluconsäure (18 mg ∼ 0,1 mmol) verwendet wurde.

Nach der Lyophilisation gelingt es nicht, die Lösung in 10 mL Wasser zu lösen, sondern es bleibt eine Suspension von Amphotericin B zurück.

### Beispiel 2

### Herstellung einer Amiodaron-Injektionslösung

10 g HES-Aldonsäure 10/0,4 werden in 30 ml Wasser für Injektionszwecke bei ca. 50 °C gelöst. Zur Lösung werden 3 g Amiodaron-Hydrochlorid gegeben und die Temperatur so lange gehalten bis sich eine klare Lösung ergibt. Danach wird mit dem gleichen Volumen einer 10 Gew. %-igen Glucoselösung in Wasser für Injektionszwecke verdünnt und die Lösung auf Raumtemperatur abgekühlt. Danach wird der pH-Wert mit Natronlauge auf 3,4 eingestellt. Nach Sterilfiltration wird die Lösung aseptisch in Ampullen abgefüllt. Die resultierende Lösung bleibt auch nach mehrmonatigem Lagern im Kühlschrank klar, transparent und partikelfrei.

### Beispiel 3

### Herstellung einer Vancomycin-Injektionslösung

510 mg Vancomycin-HCl △ 500 mg Vancomycin Base werden in 8 ml einer 25 Gew. %igen Lösung von HES 10/0,4 - Aldonsäure in destilliertem Wasser gelöst. Der pH-Wert der Lösung wird mit 0,1 N Natronlauge auf pH 7,3 eingestellt (ca. 4 ml). Dabei bleibt die Lösung klar.
Anschließend wird mit 0,2 µm Membranfilter sterilfiltriert und in Injektionsvials abgefüllt. Die resultierende Lösung bleibt auch nach mehrmonatigem Lagern im Kühlschrank klar, transparent und partikelfrei.

### Vergleichsbeispiel 3

Der Versuch wird analog zu Beispiel 3 durchgeführt, mit dem Unterschied, dass Anstelle von HES 10/0,4 - Aldonsäure HES 10/0,4 verwendet wird.
Die resultirende Lösung trübt sich nach 2-3 Stunden ein und Vancomycin fällt aus.

### Vergleichsbeispiel 4

Der Versuch wird analog zu Beispiel 3 durchgeführt, mit der Ausnahme, dass Anstelle von HES 10/0,4 - Aldonsäure destilliertes Wasser verwendet wird. Die resultirende Lösung trübt sich nach wenigen Minuten ein und Vancomycin fällt aus.

### Beispiel 4

### Herstellung einer Vancomycin-Injektionslösung

500 mg Vancomycin-Base (hergestellt aus dem Vancomycin-Chlorid durch Addition einer äquimolaren Menge an Natronlauge unter Ausfällung aus konzentrierter wässriger Lösung) werden mit 10 ml einer 20 Gew. %-igen HES 10/0,4-Aldonsäure Lösung in destilliertem Wasser versetzt.
Die Base löst sich auf (pH 7,0). Nach Sterilfiltration mit 0,2 µm Membranfilter wird in Injektionsfläschchen abgefüllt.
Die resultierende Lösung bleibt auch nach mehrwöchigem Lagern im Kühlschrank klar, transparent und partikelfrei.

## Patentansprüche

1. Molekularer Komplex aus einer hochmolekularen Trägersubstanz und einem Arzneistoft, wobei die Trägersubstanz funktionelle Gruppen aufweist, wodurch die Trägersubstanz und der Arzneistoff nicht kovalent gebunden sind, **dadurch gekennzeichnet, dass**
- der Arzneistoff in Wasser unlöslich oder schwer löslich ist,
- die Trägersubstanz in Wasser gut löslich ist,
- die Trägersubstanz ein Stärkederivat ist und nur eine funktionelle Gruppe zur nicht kovalenten Bindung des Arzneistoff in jedem Molekül aufweist, wobei die funktionelle Gruppe eine saure oder basische Gruppe ist,
- der Komplex wasserlöslich ist
- die Trägersubstanz ein gewichtsgemitteltes Molekulargewicht im Bereich von 1.000 bis 1.000.000 Dalton aufweist,
- der Arzneistoff eine funktionelle Gruppe aufweist, die Komplexbildung ermöglicht, die aus einer basischen oder einer sauren Gruppe ausgewählt ist.

2. Komplex nach Anspruch 1, **dadurch gekennzeichnet, dass** der Komplex parenteral applizierbar ist.

3. Komplex nach mindestens einem der vorangehenden Ansprüche, **dadurch gekennzeichnet, dass** das Stärkederivat die funktionelle Gruppe am vormals reduzierenden Ende aufweist.

4. Komplex nach Anspruch 3, **dadurch gekennzeichnet, dass** die saure Gruppe eine Aldonsäuregruppe ist.

5. Komplex nach mindestens einem der vorangehenden Ansprüche, **dadurch gekennzeichnet, dass** die basische Gruppe eine primäre Aminogruppe ist.

6. Komplex nach Anspruch 5, **dadurch gekennzeichnet, dass** die primäre Aminogruppe durch Konjugation eines aliphatischen Diamins mittels einer Amidbindung an eine Aldonsäuregruppe gebunden ist.

7. Komplex nach mindestens einem der Ansprüche 1 bis 4 oder 6, **dadurch gekennzeichnet, dass** das Stärkederivat HES ist.

8. Komplex nach Anspruch 7, **dadurch gekennzeichnet, dass** der Arzneistoff ausgewählt ist aus der Gruppe, bestehend aus den Polyenmakrolid-Antibiotika Amphotericin B, Nystatin und Natamycin.

9. Komplex nach Anspruch 8, **dadurch gekennzeichnet, dass** die HES-Aldonsäuren ein gewichtsgemitteltes Molekulargewicht von 1,000 bis 300.000 Dalton, einen molaren Substitutionsgrad von 0,1 bis 0,5 und ein C2/C6 Verhältnis von 2 bis 11 aufweisen.

10. Komplexe nach Anspruch 9, wobei die HES-Aldonsäuren ein gewichtsgemitteltes Molekulargewicht von 6.000 bis 16.000 Dalton, einen molaren Substitutionsgrad von 0,35 bis 0,45 sowie ein C2/C6-Verhältnis von 9 bis 11 aufweisen und der Arzneistoffs Amphotericin B ist.

11. Komplexe nach Anspruch 9, wobei die HES-Aldonsäuren ein gewichtsgemitteltes Molekulargewicht von 6.000 bis 16.000 Dalton, einen molaren Substitutionsgrad von 0,35 bis 0,45 sowie ein C2/C6-Verhältnis von 9 bis 11 aufweisen und der Arzneistoff das Antiarrhythmikum Amiodaron ist.

12. Injektionslösung, oder Infusionslösung **dadurch gekennzeichnet, dass** diese einen Komplex gemäß Anspruch 1 bis 11 aufweist.

13. Verfahren zur Herstellung von Komplexen nach Anspruch 1 bis 11, **dadurch gekennzeichnet, dass** man den Arznei stoff und die hochmolekulare Trägersubstanz in einem kompatiblen Lösemittel löst und der resultierenden Lösung das Lösemittel unter schonenden Bedingungen entzieht.

14. Verfahren zur Herstellung von Injektionslösungen oder Infusionslösung nach Anspruch 12, **dadurch gekennzeichnet, dass** man einen Komplex nach Anspruch 1 bis 11 in einer wässrigen Lösung löst.

## Claims

1. A molecular complex of a high molecular weight carrier substance and of a medicinal substance, where the carrier substance has functional groups whereby the carrier substance and the medicinal substance are bound non-covalently, wherein
- the medicinal substance is insoluble or slightly soluble in water,
- the carrier substance is readily soluble in water,
- the carrier substance is a starch derivative and has only one functional group for non-covalent binding of the medicinal substance in each molecule, wherein the functional group is an acidic or a basic group,
- the complex is water soluble,
- the carrier substance has a weight average molecular weight of from 1000 to 1 000 000 Daltons,
- the medicinal substance comprises a functional group making complex binding possible selected from the group consisting of a basic group and an acidic group.

2. The complex as claimed in claim 1, wherein the complex can be administered parenterally.

3. The complex as claimed in at least one of the preceding claims, wherein the starch derivative has the functional group at the previously reducing end.

4. The complex as claimed in claim 3, wherein the acidic group is an aldonic acid group.

5. The complex as claimed in at least one of the preceding claims, **characterized in that** the basic group is a primary amino group.

6. The complex as claimed in claim 5, **characterized in that** the primary amino group is bound to an aldonic acid group by conjugation of an aliphatic diamine by means of an amide bond.

7. The complex as claimed in at least one of the claims 1 to 4 or 6, wherein the starch derivative is HES.

8. The complex as claimed in claim 7, wherein the medicinal substance is selected from the group consisting of the polyene macrolide antibiotics amphotericin B nystatin and natamycin.

9. The complex as claimed in claim 8, wherein the HES-aldonic acids have a weight average molecular weight of from 1000 to 300 000 Daltons, a molar substitution level of from 0.1 to 0.5 and a C2/C6 ratio of from 2 to 11.

10. Complexes as claimed in claim 9, wherein the HES-aldonic acids have a weight average molecular weight of from 6000 to 16 000 Daltons, a molar substitution level of from 0.35 to 0.45 and a C2/C6 ratio of from 9 to 11 and the medicinal substance is amphotericin B.

11. Complexes as claimed in claim 9, wherein the HES-aldonic acids have a weight average molecular weight of from 6000 to 16 000 Daltons, a molar substitution level of from 0.35 to 0.45 and a C2/C6 ratio of from 9 to 11, and the medicinal substance is the antiarrhythmic amiodarone.

12. An injection solution or an infusion solution, wherein it comprises a complex as claimed in claim 1 to 11.

13. A process for preparing complexes as claimed in claim 1 to 11, wherein the medicinal substance and the high molecular weight carrier substance are dissolved in a compatible solvent, and the solvent is removed from the resulting solution under mild conditions.

14. A process for producing injection solutions or infusion solutions according to claim 12, wherein a complex as claimed in claim 1 to 11 is dissolved in an aqueous solution.

## Revendications

1. Complexe moléculaire formé par une substance support de haut poids moléculaire et une substance médicamenteuse, la substance support présentant des groupes fonctionnels, par lesquels la substance support et la substance médicamenteuse sont liées de manière non covalente l'une à l'autre, **caractérisé en ce que**
- la substance médicamenteuse est insoluble ou difficilement soluble dans l'eau ;
- la substance support est bien soluble dans l'eau,
- la substance support est un dérivé de l'amidon et ne présente qu'un seul groupe fonctionnel par molécule pour la liaison non covalente de la substance médicamenteuse, le groupe fonctionnel étant un groupe acide ou basique,
- le complexe est soluble dans l'eau,
- la substance support présente un poids moléculaire moyen en poids situé dans l'intervalle allant de 1000 à 1 000 000 dalton,
- la substance médicamenteuse présente un groupe fonctionnel permettant la formation d'un complexe, choisi parmi un groupe basique ou un groupe acide.

2. Complexe selon la revendication 1, **caractérisé en ce que** le complexe peut être administré par voie parentérale.

3. Complexe selon au moins l'une des revendications précédentes, **caractérisé en ce que** le dérivé d'amidon présente le groupe fonctionnel sur l'extrémité antérieurement réductrice.

4. Complexe selon la revendication 3, **caractérisé en ce que** le groupe acide est un groupe acide aldonique.

5. Complexe selon au moins l'une des revendications précédentes, **caractérisé en ce que** le groupe basique est un groupe amino primaire.

6. Complexe selon la revendication 5, **caractérisé en ce que** le groupe amino primaire est lié par conjugaison d'une diamine aliphatique via une liaison amide sur un groupe acide aldonique.

7. Complexe selon au moins l'une des revendications 1 à 4 ou 6, **caractérisé en ce** le dérivé de l'amidon est de l'hydroxyéthylamidon (HEA).

8. Complexe selon la revendication 7, **caractérisé en ce que** la substance médicamenteuse est choisie dans le groupe formé par les antibiotiques de type macrolides polyènes que sont l'amphotéricine B, la nystatine et la natamycine.

9. Complexe selon la revendication 8, **caractérisé en ce que** le HEA-acide aldonique présente un poids moléculaire moyen en poids allant de 1000 à 300 000 dalton, un taux de substitution molaire allant de 0,1 à 0,5 et un rapport C2/C6 allant de 2 à 11.

10. Complexe selon la revendication 9, **caractérisé en ce que** le HEA-acide aldonique présente un poids moléculaire moyen en poids allant de 6000 à 16 000 dalton, un taux molaire de substitution allant de 0,35 à 0,45 et un rapport C2/C6 allant de 9 à 11, et **en ce que** la substance médicamenteuse est l'amphotéricine B.

11. Complexe selon la revendication 9, **caractérisé en ce que** le HEA-acide aldonique présente un poids moléculaire moyen en poids allant de 6000 à 16 000 dalton, un taux molaire de substitution allant de 0,35 à 0,45 et un rapport C2/C6 allant de 9 à 11, et **en ce que** la substance médicamenteuse est l'anti-arythmique amiodarone.

12. Solution pour injection ou solution pour perfusion, **caractérisée en ce que** celle-ci contient un complexe selon les revendications 1 à 11.

13. Procédé de préparation de complexes selon les revendications 1 à 11, **caractérisé en ce que** l'on dissout la substance médicamenteuse et la substance support de haut poids moléculaire dans un solvant compatible et qu'on élimine le solvant de la solution obtenue dans des conditions douces.

14. Procédé de préparation de solutions pour injection ou d'une solution pour perfusion selon la revendication 12, **caractérisé en ce que** l'on dissout un complexe selon les revendications 1 à 11 dans une solution aqueuse.
